# EUROPEAN PATENT APPLICATION

(11) **EP 4 339 612 A1**
(43) Date of publication of application: **20.03.2024**
(21) Application number: 23197992.3
(22) Date of filing: 18.09.2023
(51) Int. Cl.: G01N 33/18, B63B 35/00, G01V 9/00

(54) **A METHOD OF OBTAINING SAMPLES FROM A BODY OF WATER AND A MARINE VESSEL**

(30) Priority: 19.09.2022 EP 22196344
(71) Applicant: DanaDynamics ApS, 5700 Svendborg (DK)
(72) Inventor: Møller, Lasse Skriver, Svendborg (DK); Stockholm, Martin, Svendborg (DK)
(74) Representative: Dragsted Partners A/S

(57) **Abstract**

In accordance with the invention, there is provided a method of obtaining samples from a body of water by a marine vessel in a body of water the marine vessel comprising an electronic device, the method comprising: obtaining a predefined model data of a first water sample device, generating a first primary control signal for the first water sample device and performing a first movement of a first testing part in a first position in the body of water, receiving a first sensor data from the first water sample device, generating first comparison data where the first sensor data is compared with the predefined model data of the first water sample device, generating a second primary control signal to the first water sample device based on the first comparison, performing a second movement of the first water testing part relative to the body of water.

## Description

### Technical Field

A method of obtaining samples from a body of water by a marine vessel in a body of water the marine vessel comprising an electronic device.

### Description

Environmental regulations have been set in certain parts of the world where it is necessary for governments, municipalities and/or harbours to monitor the water quality in water environments, such as lakes, coastlines and harbours. Collection of water samples and testing the water is a very time-consuming process, which requires expensive equipment and a plurality of operators in order to collect samples in correct positions as well as at correct depths.

Thus, when the water samples are collected or where sensors are used to measure attributes of the water locally, there are several considerations that have to be made, such as ensuring the correct geographic tagging of the samples, as well as identification of the conditions under which a water sample has been taken. Traditionally this is done by manual labour, where a user sails along a coastline or in a body of water in order to collect samples and test water in predefined locations. Such a process is very labour intensive and time consuming, as the testing has to be done in a predefined manner in order to ensure that the samples and the tests are taken at the correct positions, as well as at the correct depths, in order to ensure that the samples and tests are representative of the true conditions in the body of water.

Thus, there is a need to improve the testing and sampling procedures, where it may be possible to objectively identify whether the sample and/or the testing of the water fulfils the requirements of the testing regulations, or at least to increase the likelihood that the samples or tests are useable.

In accordance with the invention, there is provided a method of obtaining samples from a body of water by a marine vessel in a body of water the marine vessel comprising an electronic device, the method comprising: obtaining a predefined model data of a first water sample device, generating a first primary control signal for the first water sample device and performing a first movement of a first testing part in a first position relative to the the body of water, receiving a first sensor data from the first water sample device, generating first comparison of data where the first sensor data is compared with the predefined model data of the first water sample device, generating a second primary control signal to the first water sample device based on the first comparison, performing a second movement of the first water testing part relative to the body of water.

Within the context of the present disclosure the term "marine vessel" may mean any vessel that is capable of navigating in a body of water, on the surface of the body of water, or possibly submerged in the body of water, and may move from a geographical first position to a geographical second position of the body of water. Thus, a marine vessel may be any kind of watercraft.

The term "obtaining samples" may mean either to obtain physical samples of water from a body of water or may mean that measurements are taken of samples of water where the samples are part of the body of water and are not physically taken from the body of water. Thus, obtaining samples may mean that a predefined amount of water is collected from the body of water in a container, or may mean that a measurement is taken of a sample of water, such as translucency measurement made in the body of water. Furthermore, the term "obtaining samples" may be a measurement of a body of water where the measurement may be taken at a first distance from the body of water. Such a measurement may e.g. be a measurement taken above the surface of the body of water, where the measurement device is not in contact with the body of water, and where the measurement device may divert optical waves, electronic signals, radio signals or other types of signals that are capable of penetrating the body of water or are capable of measuring a property of the surface of the body of water.

Within the context of the present disclosure, the term first movement, and/or second movement may mean a first action and/or second action or any subsequent action, where the action may be a physical movement, physical action or an electronic action of the water testing part. The first and/or second action may also be understood as a first and/or a second sample action, where a testing device samples the water with a physical action of the first testing part, or may be in the form of an electrical action where a sensor is activated to perform an action.

The predefined model data of a first water sample device, or any subsequent water sample device, may be in the form of instructions on how the water sample device is to be utilised to obtain a sample or to make an in situ test of the body of water. The predefined model data may include instructions of a certain procedure on how to obtain a water sample. The predefined model data may include instructions on for how long a certain test should be performed and in how many iterations the testing should be done. An example may be that a translucency test should be performed in iterative steps, where the first testing part is submerged at a predefined depth, and an optical device (camera) observes if the first testing part is visual at the predefined depth. If it may be detected by the optical device, the first testing part may be submerged at a second depth, and this can be repeated until the optical device is not capable of detecting the first testing part. In this case, the first testing part may be an object having a physical structure that is to be viewed by an optical device, where the optical device may be the sensor and may provide sensor data for the testing.

The predefined model data may be a first water sample device or any subsequent water sample device in the form of instructions on how the water sample device is to be used to measure a predefined quantity of the body of water. The water sample device may e.g. be an optical camera that is pointed towards the surface of the body of water, or may be an optical and/or a thermal camera that may be instructed to perform a visual inspection of a predetermined part of the body of water.

The sensor may also be a temperature sensor, pressure sensor or other types of sensors that may be attached to the first testing part, and when the first testing part is submerged into the body of water, the sensor will provide a first sensor data.

When the sensor has provided a first sensor data, the sensor data is compared to predefined model sensor data in the predefined model data and a first comparison data is generated. The predefined model data may also include information on the testing procedure, testing conditions, or any other type of data that may be relevant to the water testing of the water sample device. Each testing device may have a predefined model data, where the predefined model data comprises specific information relating to each testing device, and instructions and/or information on how the testing should be performed, and possibly conditional instructions on how to proceed with testing, should the obtained sample not include the necessary information or only parts of the necessary information. Furthermore, the predefined model data may also include instructions on when the testing procedure is finished, where the comparison data is utilised to confirm or deny whether the testing procedure for a specific testing device is completed. If the comparison data shows that the data is not sufficient, the model data may include instructions on how the water sample device should perform the next test to obtain a new comparison data.

The second primary control signal is sent to the first water sample device based on the first comparison, using the comparison data to determine the next step of the first water sample device. The second primary control signal may be utilised to instruct the first water sample device that the testing is complete, and the signal will terminate the testing procedure (obtaining sample procedure) for the first testing device, thus the second movement of the first water testing part may be retrieved from the body of water or may be moved out of the way so that a second water sample device may be arranged in a position to initiate a second (different) testing procedure. The second primary control signal may be generated based on the comparison data where the comparison data indicates that more sensing data may be needed to complete the testing procedure, where the second movement may be to reintroduce the first testing part into the body of water, or may send the first water testing part to a different depth in the body of water, or may move the second testing part to a different part of the body of water to perform a second test (obtain sample) and obtain a second sensor data. This procedure may be repeated until the comparison of the sensor data and model data indicates that the testing is complete for the specific water sample device.

Thus, the method for obtaining samples from a body of water may automatically initiate and complete a testing cycle of a first water sample device, where the model data indicates, based on the comparison of the sensor data, whether the testing is complete, and the marine vessel may be directed to a second position to perform a new procedure of obtaining samples from the body of water.

The present method may also be utilized to:
In one exemplary embodiment of the present disclosure, the performance of the first movement of a first testing part may be in a first position in the body of water, and/or where the performance of the second movement of a second testing part may be in a second position in the body of water, and/or where the performance of a movement of any testing part in any position may be in the body of water. Thus, the testing part may be submerged at last partly into the body of water, where the first position is at least partly submerged, and where the movement to a second position may also be at least partly submerged. Alternatively, the movement may be to move the testing part from an at least partly submerged position to a position where the testing part is not in contact with the body of water.

In one exemplary embodiment of the present disclosure, the first comparison data may be used to update the predefined model data of the first water sample device. The first comparison data and/or the first sensor data may be utilised to update the predefined model of the first water sample device. The predefined model data may comprise model data that has been generated by a pattern recognition system, where the pattern recognition system may be based on the sensor signals, predefined limits for the testing procedure, data reviewed by humans in the form of statistical analysis or other types of verification of the data of the model or may be based on predefined rules and instructions. The comparison data and/or the sensor data may be utilised to optimise the pattern recognition algorithm in the system, where the pattern recognition system may utilise machine learning (artificial intelligence) for pattern recognition, where the sensor data may be utilised to build or to continue to build on the models, where training data may have been utilised to initiate the data, and where the real sensor data may be utilised to optimise predictions or decisions of the first water sample device, and/or for the control of the marine vessel.

In one exemplary embodiment of the present disclosure, the first comparison data may be configured to determine the second movement of the first water testing part relative to the body of water. The first comparison data may be processed by a computer processor to assist in performing the next task of the first water sample device. The next task may be a further testing task or may be a retrieval of the first testing part from the body of water. The comparison data may be utilised to identify which movement the testing part is supposed to perform, as the testing procedure can vary from one water sample device to another water sample device. One water sample device may comprise a water testing part which is introduced into the body of water to collect a sample of water into a container, where the sensor of the water sample device may e.g. be configured to determine whether there is a water sample inside the container. Alternatively, another water sample device may be a device configured to test the pH level of the water in a certain position, where the first testing part introduces a pH tester into the body of water, where the model data indicates that it may be necessary to test the pH level in a second position of the body of water, where the second position may be at a greater depth of water. Thus the second movement of the water testing part may be to introduce the testing device to a second depth of water, where the sensor data may indicate the pH value at the second depth. Thus, the second sensor data may also indicate the depth of the water sample, and when the second sensor data has collected, the second sensor data may be used to determine that the testing procedure for the water sample device is complete.

In one exemplary embodiment of the present disclosure, the predefined model data of a first water sample device, or any subsequent water sample device, may be in the form of instructions on how the water sample device is to be used to measure a predefined quantity of the body of water. Such measurement may e.g. be performed in the form of a UAV (Unmanned Aereal Vehicle) or a ROV (Remotely Operated Underwater Vehicle), where the UAV or the ROV may be a water sample device in accordance with the present application. The UAV or ROV may be remotely operated, or may have predefined instructions on how to perform a sampling of the body of water. An example of such instructions, may e.g. be to instruct an ROV to submerge to a predefined position in the body of water, where the ROV is instructed to obtain optical and/or thermal images of e.g. the surface of the seabed of the body of water, or to send radio frequencies to the bottom. This may be utilized to perfom a more detailed measurement of the body of water, to identify e.g. a position of an electric cable that is arranged on the seabed, or to perform a physical action in the body of water.

In one embodiment the ROV may be seen as a water sample device that may perform measurements with a sonar, laser scanner, acoustic michrophones, flow measurements, magnetometer, ultrasoundsensor, or a senser that may be able to "sniff" or to perform measurements of the environment which the ROV has been deployed towards. The ROV may also be utilized to carry sensors for measurements of Rugged Dissolved Oxygen (RDO), Temperature, Conductivity, pH/ORP, Turbidity, Chlorophyll a, Phycocyanin (BGA-PC), Phycoerythrin (BGA-PE), FDOM, Crude Oil, Rhodamine WT, Fluorescein WT, Ammonium (ISE), Chloride (ISE) or Nitrate (ISE).

A ROV and/or a UAV may be provided with wireless and/or wired communication with the marine vessel, where the marine vessel may provide the ROV and/or the UAV with instructions while the ROV and/or the UAV are being deployed from the marine vessel.

The marine vessel may be provided with a UAV and/or a ROV, where the UAV and/or the ROV may be provided with a water sample device to perform a measurement on the body of water in one or more positions, where the UAV and/or the ROV may perform a measurement in a first position, and where the UAV and/or the ROV may be perform a subsequent measurement in a subsequent position relative to the body of water.

In one exemplary embodiment of the present disclosure, the second movement of the first water sample device may be retrieving the first water testing part from the body of water. The comparison data may be utilised to identify which movement the testing part is supposed to perform, as the testing procedure can vary from one water sample device to another water sample device. The comparison data may indicate that the testing procedure for the specific water sample device is complete, and that the comparison of the model data and the sensor data indicates that all the necessary information has been obtained using the water sample device. Thus, the second movement may be to retrieve the first water testing part from the body of water to complete the testing procedure of the specific water testing part. Thus, when the specific water sample device has completed its task, a subsequent water sample device may be activated, and the method of the present disclosure may be repeated to perform a second run of a method of obtaining samples from a body of water.

In one exemplary embodiment of the present disclosure, the second movement of the first water testing part may be to perform a second movement of the first testing device to a second position relative to/in the body of water, where the second position may be different from the first position, receiving a second sensor data from the first water sample device. The second sensor data may be treated in the same way as the first sensor data, where the second sensor data may be compared with the predefined model data, and where the data comparison may be used generate a third primary control signal to the water sample device and to instruct the device to perform a third movement of the first water testing part. By moving the water testing part to a second position, the water sample device may perform a second sampling of the water where the second position may be different in depth or at a different geographical position (longitude, latitude) to obtain a more complete sampling of the body of water. The second position may either be predefined by the testing protocol (predefined model) of the water sample device or may be performed due to an incomplete, inconclusive or possibly erroneous test in the first position of the water testing part relative to the body of water.

In one exemplary embodiment of the present disclosure, the method may further comprise providing a predefined model data of a second water sample device, generating a first control signal for the second water sample device and performing a first movement of a second testing part in a first position in the body of water, receiving a first sensor data from the second water sample device, generating first comparison data where the first sensor data may be compared with the predefined model data of the second water sample device, generating a second primary control signal to the second water sample device based on the first comparison, performing a second movement of the second water testing part relative to the body of water. This means that the method may be utilised to operate at least two water sample devices, where upon completion of the method when operating the first water sample device, the method may continue and initiate the operation of a second testing device, where the second water sample device may be a water sample device that obtains different samples or tests from the body of water. The second movement of the first testing device may be to retrieve the first testing part, where the method allows the controller to determine that the testing of the first device is concluded, and based on a predefined testing procedure, the method may initiate the second testing device or may initiate the second testing device based on the results of the water sample device. Thus, the method may include a testing protocol model, where the testing protocol model may define which tests to be conducted, and in what order the testing is to be performed. The controller may receive or generate the comparison data of the first testing device and may, based on a predefined order or by the outcome of the first tests, define which water sample device is to be initiated subsequent to the first testing device. The marine vessel may comprise a plurality of water sample devices, where each water sample device may obtain samples from the body of water or test results based on sensor data, where each water sample device may have a different purpose than the other water sample devices, or may perform a test in a different way, should the sensor data of a first test indicate that a certain test cannot be performed up to a sufficient standard, where such a determination may be made by the controller on basis of the sensor data of a water sample device.

In one exemplary embodiment of the present disclosure, the method may further comprise generating a first secondary control signal for the marine vessel, where the first secondary control signal may be used to perform a positional movement of the marine vessel relative to the body of water. The first secondary control signal for the marine vessel may be a control signal that indicates that the marine vessel is to be manoeuvred from a first geographical position to a second geographical position. The first secondary control signal may be generated by a controller and may be received by a control unit of the marine vessel, where the control unit may be used to control the propulsion, depth and/or the direction of the marine vessel. Thus, if the marine vessel is to perform water testing in a plurality of geographical positions of the body of water, the first secondary control signal and any subsequent secondary control signal may be utilised to manoeuvre the marine vessel to a different geographical position, where a second sample of water may be obtained from the body of water.

In one exemplary embodiment of the present disclosure, the model data may comprise a first data set comprising a testing procedure (and thereby a first movement and a second movement of the testing part, and/or positional movement of the marine vessel) of a first testing device, second testing device and/or any subsequent testing device or a second data set comprising predefined model data for sensor data comparison. The first data set may be a data set that is predefined for any specific water sample device, where the data set may set out any procedure for performing a test using the water sample device. Thus, the data set may include any instructions on where and how the water test is to be performed, how many sensor results are necessary to have a reliable set of data, and when the testing is to be performed. The second data set may comprise comparative data where the comparative data may be training data, example data, ranges for valid results, ranges for invalid results and other types of sensor data that may assist in defining whether the water sample device has performed the necessary tests and if the tests are within an acceptable range. Thus, the second data set may comprise test results from previous tests made by the water sample device. The first data set and/or the second data set may be utilised to train a pattern recognition algorithm, e.g. a machine learning algorithm, in order to increase the likelihood that the tests made in the body of water are valid, and that the testing may be done automatically using the method of the present disclosure using an electronic device with at least one processor.

In one exemplary embodiment of the present disclosure, the first sensor data may be sensor data from one or more of conductivity sensor, temperature sensor, pressure sensor, dissolved oxygen sensor, fluorescence sensor, ORP, turbidity sensor, Voltage sensor, Photosynthetically Active Radiation (PAR) sensor, water sampler sensor, sediment core sampler sensor, Multibeam sonar sensor, Echo sounder sensor, Underwater camera sensor, Manta net sensor, Micro plastic filter pump sensor, Metal detector, Microphone sensor, optical sensor, odour sensor, rain sensor, light sensor, weight sensor, and/or cooling sensor.

In one exemplary embodiment of the present disclosure, the first water sample device, the second water sample device or any subsequent water sample device performs a measurement where the first sensor data represents attributes of at least part of the body of water. The measurement may be performed in situ where the sensor senses an attribute of the water sample to be obtained. Thus, the sensor may measure a specific attribute of the water, such as pH, viscosity, temperature, pressure, and/or other useful attributes. This type of testing means that the water is tested in situ in the body of water, where the water is not removed from the body of water, but where actual conditions of the water sample is tested. This means that the water testing part may be a part of the testing apparatus, where the water testing part may comprise a sensor that is submerged into the body of water or may comprise a part which may e.g. be optically detected, visually detected, sonically detected by a sensor that is arranged on or close to the surface of the body of wate, such as a measurement of translucency of the body of water.

In one exemplary embodiment of the present disclosure, the first water sample device, the second water sample device or any subsequent water sample device collects a part of the body water in one or more sample containers. This means that the water sample device may be configured to collect a part of the water into a container, where the container may be stored abord the marine vessel, and where the sensor may be configured to assess whether or not the container holds a sufficient amount of sample fluid taken from the body of water. The water sample device may comprise a plurality of containers that may subsequently be loaded to the first testing part, so that when a container having a sample has been retrieved to the marine vessel, the full container may be stored and an empty container may be applied to the water sample device. Thus, the stored container may be transported to a user, where further testing of the water sample may be done in laboratory settings.

The water sample devices may collect positions and/or condition data of each of the samples taken, either where water is measured in situ or taken aboard the marine vessel, where the positional data may indicate a plurality of data that indicates when, where, how, at which conditions, etc. the samples have been taken by the water sample device. Thus, the position and/or condition data may be stored in a memory, and the data may be attributed to a certain sample data set, identifying the sample which the data belongs to.

In one exemplary embodiment of the present disclosure, the method may comprise providing a first control data, where the control data may be compared to a predefined set of control data, and if the control data may be in accordance with the predefined set of control data, the method as described abovemay be initiated. By providing a first control data it may be possible to instruct the marine vessel to initiate the method of obtaining samples at predefined positions, time, temperatures or any other conditions, in order to ensure that the operators and/or the users of the marine vessel are capable of setting predefined conditions for performing the method in accordance with the present disclosure. Thus, an operator may transmit the first control data to the electronic device, where the first control data may comprise predefined coordinates where the testing is to be performed. The control data may be provided via positional systems, such as GPS, map data, or other types of positional sensors. The control data may also include wind speed data, wave data or other types of data that represent the conditions surrounding the marine vessel. Thus, when the control data is in accordance with the predefined control data, the method of obtaining samples may be initiated, and the control data may further be utilised to maintain the correct position of the marine vessel during the obtaining of the samples.

The present disclosure also relates to a marine vessel configured for obtaining samples from a body of water, the marine vessel comprising a processor, memory and an interface, wherein the processor may be configured to provide predefined model data of a first water sample device, generating a first primary control signal for the first water sample device and performing a first movement of a first testing part in a first position relative to/in the body of water, receiving a first sensor data from the first water sample device, generating first comparison data where the first sensor data may be compared with the predefined model data of the first water sample device, generating a second primary control signal to the first water sample device based on the first comparison, performing a second movement of the first water testing part relative to the body of water.

In one or more exemplary embodiments of the present disclosure, the method may be utilised in conjunction with an autonomous marine vessel. The operation of the autonomous marine vessel may include a computer system for controlling the operation of the autonomous marine vessel. The computer system may include a control module, where the control module may operate in accordance with a controller, which may include one or more processors having short-term and/or long-term data storage, storage device and instructions stored in a memory that can carry out the operations of the controller when the instructions are executed. The controller may receive data representing a desired task, where the desired task may include operation of the autonomous marine vessel in a predefined area of an electronic map. The testing may be utilised to adapt the operation of the marine vessel in a predefined area of the electronic map. In an alternative embodiment, the controller may receive data representing a desired task, where the desired task may include instructions to travel from point A to point B of the autonomous marine vessel in an area of the electronic map to perform a testing or sampling of water. The risk parameter of the first and/or the second cell may be utilised to adapt the operation of the marine vessel in defining the route from Point A to Point B of the electronic map.

In an embodiment, the controller may receive data representing a desired output. The desired output typically includes a velocity, e.g. a speed and a heading. The desired output can be based on, for example, data received from a planning module. In accordance with the desired output, the controller produces data usable as a throttle input and a steering input. The throttle input may represent the magnitude in which to engage the throttle (e.g., acceleration control) of the marine vessel to achieve the desired output. In some examples, the throttle input also includes data usable to engage a deceleration control of the marine vessel. The controller may receive a steering input which represents a steering angle, e.g. the angle at which the steering control (e.g. steering wheel, steering angle actuator, or other functionality for controlling steering angle) of the marine vessel should be positioned to achieve the desired output. The speed and the velocity of the marine vessel may be controlled by comparing positional data and/or directional data which may be received from positioning systems, such as a GPS system.

In one or more exemplary embodiments, the map data is marine chart data. The marine chart data may be in the form of an electronic nautical/marine chart having information on marinas, docks, boat ramp locations, depth levels, underwater structures, tidal information, shipping lanes, sailing lanes and other relevant information. The map data may be in the form of a graphic representation of a sea area and adjacent coastal regions, and it may show depths of water and heights of land (topographic map) natural features of the seabed, details of the coastline, navigational hazards, locations of natural and human-made aids to navigation, information on tides and currents, local details of the Earth's magnetic field, and man-made structures such as harbours, buildings and bridges.

In one or more exemplary embodiments, the control of the marine vessel may further comprise the step of receiving a first data parameter, where the risk assessment is further determined using the first data parameter. The first data parameter may be obtained from GPS, Ship Log, Weather Data or AIS transponder.

The automatic identification system (AIS) is an automatic tracking system that uses transceivers on ships and is used by vessel traffic services (VTS). When satellites are used to detect AIS signatures, the term Satellite-AIS (S-AIS) is used. AIS information supplements marine RADAR, which continues to be the primary method of collision avoidance for water transport. Although technically and operationally distinct, the ADS-B system is analogous to AIS and performs a similar function for aircraft. Information provided by AIS equipment, such as unique identification, position, course, and speed, can be displayed on a screen or an electronic chart display and an information system (ECDIS). AIS is intended to assist a vessel's watch-standing officers and allow maritime authorities to track and monitor vessel movements. AIS integrates a standardised VHF transceiver with a positioning system, such as a Global Positioning System receiver, with other electronic navigation sensors, such as a gyrocompass or rate-of-turn indicator. Vessels fitted with AIS transceivers can be tracked by AIS base stations located along coast lines or, when out of range of terrestrial networks, through a growing number of satellites that are fitted with special AIS receivers which are capable of deconflicting a large number of signatures.

In one or more exemplary embodiments, the first sensor may be one or more of LiDAR, RADAR, optical camera, FLIR Camera, Multibeam Sonar, Ultrasound and Acoustic Microphone.

As will be appreciated by one skilled in the art, the aspects of the present invention may be embodied as a system, method or computer program product. Accordingly, aspects of the present invention may take the form of an entirely hardware embodiment, an entirely software embodiment (including firmware, resident software, micro-code, etc.), or an embodiment combining software and hardware aspects that may all generally be referred to herein as a "circuit," "module," or "system." Furthermore, aspects of the present invention may take the form of a computer program product embodied in one or more computer readable medium(s) having computer readable program code embodied thereon.

### Brief description of the drawings

The following is an explanation of exemplary embodiments with reference to the drawings, in which:
Fig. 1 is a schematic perspective view of a marine vessel in accordance with the present d isclosu re,
Fig. 2 is a schematic top view of a marine vessel in accordance with the present disclosure,
Fig. 3 is a schematic view of a control system for a water sample system on a marine vessel, and
Fig. 4 is a schematic view of a method of obtaining a sample of water from a body of water according to the present disclosure.

### Detailed description

Various exemplary embodiments and details are described hereinafter, with reference to the figures when relevant. It should be noted that the figures may or may not be drawn to scale and that elements of similar structures or functions are represented by reference numerals throughout the figures. It should also be noted that the figures are only intended to facilitate the description of the embodiments. They are not intended as an exhaustive description of the disclosure or as a limitation on the scope of the disclosure. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

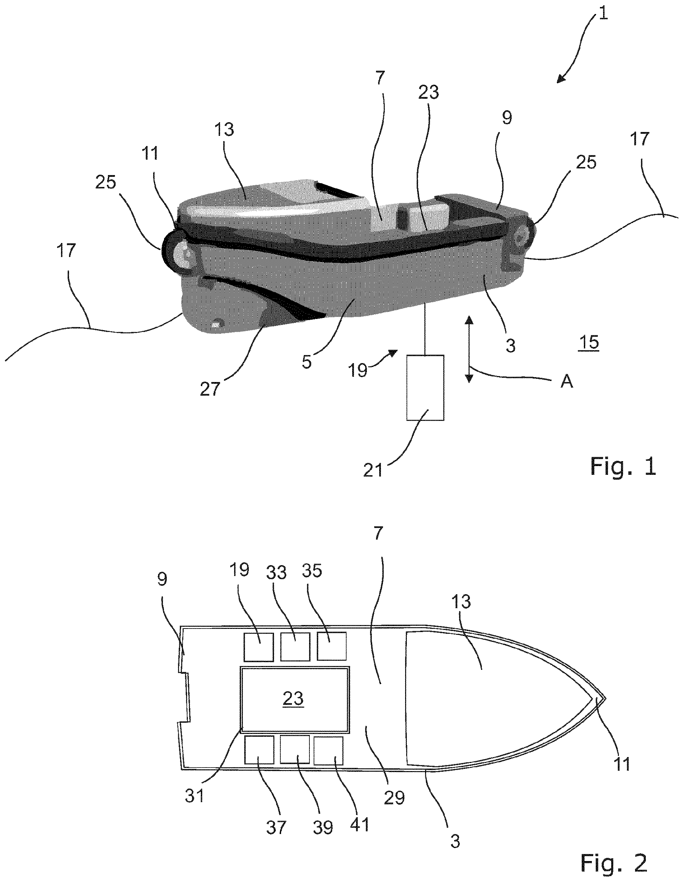

Fig. 1 shows a schematic perspective view of a marine vessel 1, where the marine vessel 1 comprises a hull 3, an outer surface 5 of the hull and the inboard volume 7 of the hull 3. The hull comprises a stern 9 and a bow 11. The marine vessel comprises a propulsion system (not shown) and a control unit (not shown) where the control unit is configured to provide control input to the propulsion system and a directional control of the marine vessel 1. The marine vessel may be arranged on a body of water 15, where the marine vessel floats on the surface 17 of the body of water 15.

The marine vessel 1 may comprise an inboard housing 13, where the inboard housing 13 may comprise an electronic device, such as a processor and/or a computer system, where the electronic device is positioned inside the inboard housing 13 in order to protect the electronic device from weather conditions, sea conditions or any external conditions that may affect a marine vessel that is arranged on a body of water.

The marine vessel may comprise at least one water sample device 19, where the at least one water sample device 19 may comprise a water testing part 21, where the water testing part may be selectively submerged into the body of water 15, in order to obtain samples from the body of water or to perform a test of a part of the body of water 15. The water testing part 21 may be configured to be selectively submerged and retrieved from the body of water in the direction A. The marine vessel may comprise a set of retractable wheels 25, where the wheels 25 may be retracted into a sailing position, as shown in Fig. 1, and may be extended into a land position, where the wheels extend below the lowest part of the bottom 27 of the hull 3, allowing the wheels to suspend the marine vessel 1 from land surface and allow the marine vessel to be rolled via the set of wheels 25 on land. Thus, the marine vessel may be an amphibious boat, which may be driven or towed on land, while also being able to travel on a body of water 15.

Fig. 2 shows a top schematical view of the marine vessel 1 seen in Fig. 1, where the same reference numbers used in Fig. 1 apply also to Fig. 2. The inboard volume 7 of the hull 3 may comprise a moon-pool 23, where the moon-pool 23 provides access to the body of water 15 inboard the hull 3 of the marine vessel 1. The moon-pool may be an opening in the base 29 of the hull 3 which may have an inner wall 31 that ensures that the water in the moon-pool 23 does not enter the inboard volume 7 of the hull. Thus, the moon-pool 23 may provide an access for one or more water sample devices 19. The water sample devices 19 may at least partly be mounted above the moon-pool 23, where a water testing part 21 of the water sample device 19 may be introduced into the body of water 15 via the moon-pool 23. Alternatively and/or additionally, the water sample devices 19 may be mounted around the periphery of the inner wall 31 of the moon-pool 23, where a second water sample device 33, a third water sample device 35 and subsequent water sample devices 37, 39, 41 may be positioned, to provide access for a water testing part 21 of the water sample devices 19 into the body of water 15 via the moon-pool 23.

The marine vessel may comprise a control unit 43 configured to control the propulsion and directional equipment (not shown) of the marine vessel 1 where the control unit is capable of receiving control signals from a controller 45 that provides secondary control signals to control the geographical position or geographical navigation of the marine vessel on the body of water 15. The control unit may be in electrical communication with the propulsion and directional equipment of the marine vessel and may be in electrical communication with the controller 45.

The controller 45 may be in electrical communication with the water sample devices 19, 33, 35, 37, 39, 41, where the controller may provide primary control signals to the water sample devices 19, 33, 35, 37, 39, 41 to provide control input to perform tests on the body of water. The water sample devices may have one or more actuators (not shown) that are configured to submerge and retrieve at least one water testing part into the body of water 15.

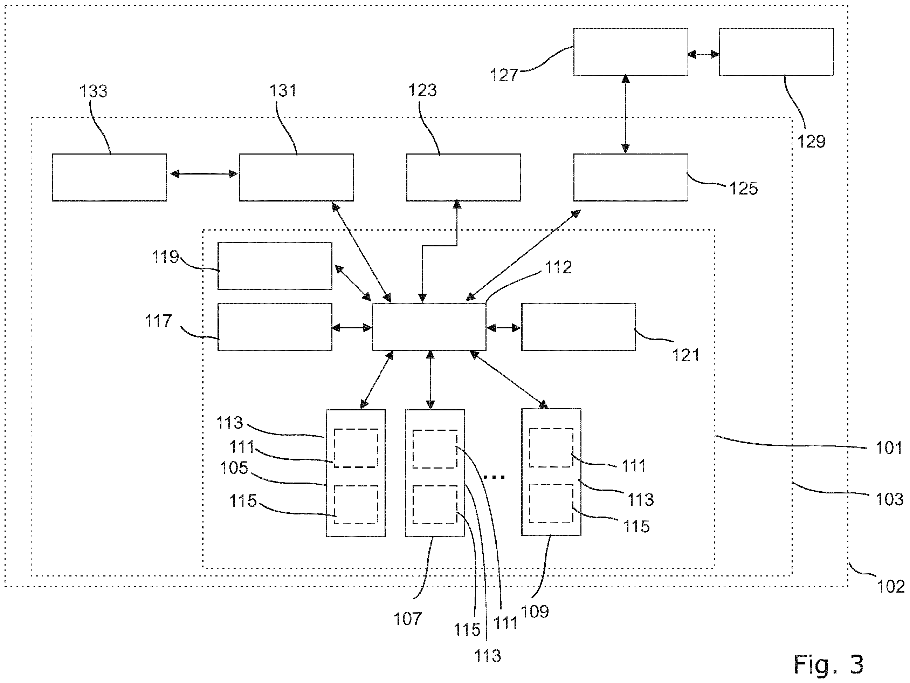

Fig. 3 is a schematic view of a control system 101 for a water sample system 102 on a marine vessel 103, having a first water sample device 105, a second water sample device 107 and a subsequent water sample device 109. The control system 101 may comprise a controller 112 which is in electrical communication with the first water sample device 105, second water sample device 107 and any one or more subsequent water sample devices 109. The control system is configured to perform the method of obtaining samples from a body of water in conjunction with the water sample devices 105, 107, 109. The controller 112 is configured to receive a predefined model data 111 from the first water sample device 105, where the predefined model data may define the testing procedure of the water sample device 105. The controller 112 will generate a first primary control signal for the first water sample device 105 where a first water testing part 113 of the first water sample device 105 will move into a first position in the body of water. The first water sample device 105 comprises a first sensor 115, where the first sensor will perform a measurement of an attribute of the water, and/or where the sensor will detect whether the test has been correctly performed. The first sensor data from the first sensor 115 will be compared with the model data 111, and where the controller 112 will generate a second primary control signal to the water sample device based on the first comparison. Subsequently, the first water testing part may perform a second movement in the body of water. The second water sample device and the subsequent water sample devices may also have a predefined model data 111, a water testing part 113 and a sensor 115.

The first water sample device may be a water sample device 105 that is configured to lower a sensor via the first testing part 113 into the water or may be a sensor that is positioned above the surface of the body of water and may sense the position of the first water testing part to identify a certain state of the body of water, such as the level of translucency of the body of water.

The second testing device 107 and a plurality of subsequent sample devices 109 may be configured to measure different attributes of the body of water than the first 105 and/or second 107 water sample devices, where the controller 112 may be configured to initiate the water testing procedure of each device, either simultaneously with another water sample device or subsequent to another water sample device.

Each water sample device 105, 107, 109 may have their specific predefined model data 111 that is specifically adapted to work with the specific type of sensor 115, where the controller 112 may also be configured to adjust the model data 111 based on the testing being performed by the sensors 115. Thus, the predefined model data may be updated in accordance with the results of the measurements, where the updating may be utilised to improve the reliability of the testing, as the testing may be done autonomously without any intervention by human operators.

The sensor 115 may either be part of the water testing part 113 and may be submerged together with the water testing part into the body of water or may be separated from the water testing part 113, where the water testing part 113 is submerged into the water, while the sensor positioned distal to the water testing part and possibly above the waterline of the body of water.

The controller 112 may be in electrical communication with a pattern recognition system 117, where the pattern recognition system may be utilised to optimise the testing procedure and/or the predefined model data 111 of the water sample devices 105, 107, 109.

The controller 112 may further be in electrical communication with a test protocol 119 where the test protocol may comprise data on how to perform the testing procedure using a marine vessel, or may comprise a testing procedure or information on how to perform a testing protocol using a first testing device, a second testing device or a futher testing device. Thus the test protocol may comprise information on which tests are to be performed by the marine vessel, and/or in which order the testing protocol is to be performed, and/or may also indicate how a specific water testing device is to perform tests in a certain situation and/or instruction on how the testing is to be performed to obtain a useable water sample for measuring a predefined attribute of the body of water.

The controller 112 may further be in electrical communication with a navigational system 121 where the navigational system may be a pattern recognition system for a navigational system, where the controller 112 may further communicate with navigational information such as a navigational chart 123. The marine vessel may be provided with a plurality of sensors that can update actual conditions, using the navigational system 121 to identify obstacles or other marine vessels that are not represented on the geographical and/or navigational chart 123.

The controller 112 may further be in electrical communication with a communication system 125, where the communication system may be capable of communication with the cloud and/or the internet 127. A user terminal 129 may be in communication with the communication system 125 via the cloud and/or the internet 127, where the user terminal 129 may be used to provide tertiary control signals to the controller 112 to adjust the parameters of the water sampling procedure, and/or to override the testing procedures, and/or to monitor the process of the water testing procedure, or may communicate with the water sample device and/or the marine vessel in any suitable form to provide input or output from the marine vessel 103 and/or the water sample system 102 and/or the control system 101.

The controller 112 may be in electrical communication with a control unit 131, where the control unit 131 may be connected to the propulsion and/or drive and/or steering devices 133 of the marine vessel. Thus, the controller 112 may, based on predefined model data, navigational information, adjust the geographical position of the marine vessel 103 and/or maintain the geographical position of the marine vessel during the operation of the testing procedure of the marine vessel.

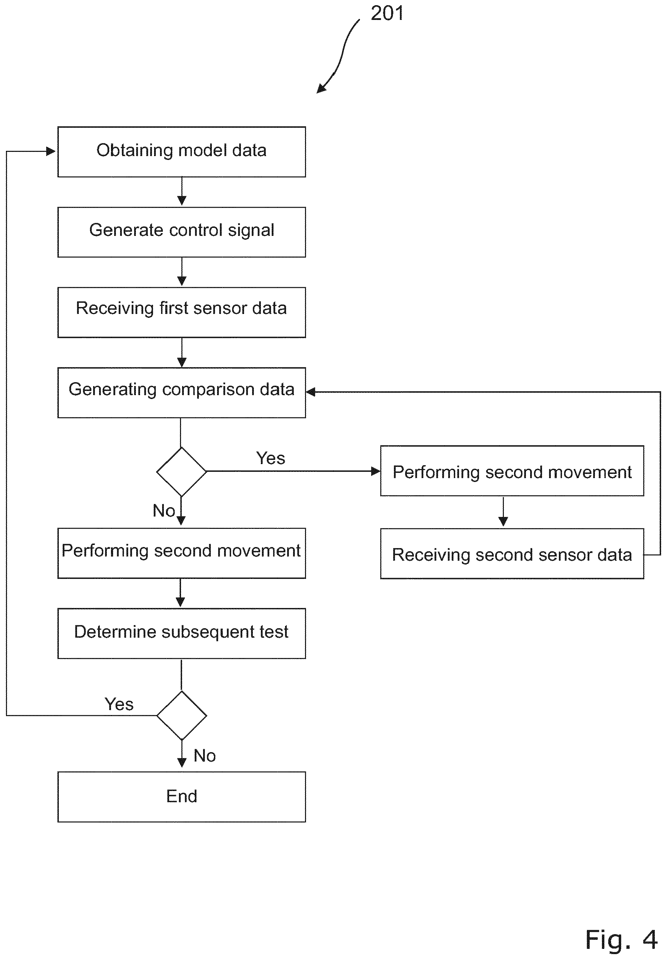

Fig. 4 is a schematical view of a method 201 for obtaining a sample of water from a body of water according to the present disclosure. The method is initiated by obtaining model data for a specific water sample device. The model data is utilised to generate a primary control signal for the water sample device and/or the water testing part and to move the water testing part into its first position. When the movement has been completed, the sensor measures the specific attribute of the body of water, and the sensor data is received by an electronic device (controller). The sensor data is used to compare with the predefined model data in order to generate a comparison data. The comparison data may be utilised to determine the next movement of the water testing part, where the comparison data is used to determine whether the water sample device has obtained the necessary information from the water sample. If testing using the first water sample device needs further samples, the decision follows the process in the direction of the yes and where a second movement of the first testing part is performed, a second sensor data is received. The second sensor data is utilised to generate comparison data, and the loop continues. When the loop is finished, and the comparison data indicates that no further testing is required, the decision process follows the process in the direction of no, and the second movement may be to perform a second movement to retrieve the first testing part.

When the first testing part has been retrieved, the controller may determine whether or not there are further water sample devices that have to be utilised to measure attributes of the body of water. If further sampling is needed, the process returns to its initial step of obtaining model data for the second water sample device and continues with the process as discussed above. When it is confirmed that all water sampling has been completed with all water sample devices, the controller will end the process, and follow the decision process to the end. The end may mean that the controller will manoeuvre the marine vessel to a second geographical position of performing subsequent tests in the body of water or may return back to its operator to complete the water testing procedure.

The use of the terms "first", "second", "third" and "fourth", "primary", "secondary", "tertiary" etc. does not imply any particular order but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims.

Although features have been shown and described, it will be understood that they are not intended to limit the claimed invention, and it will be made obvious to those skilled in the art that various changes and modifications may be made without departing from the spirit and scope of the claimed invention. The specification and drawings are, accordingly, to be regarded in an illustrative rather than restrictive sense. The claimed invention is intended to cover all alternatives, modifications, and equivalents.

The title, background, brief description of the drawings, abstract, and drawings are hereby incorporated into the disclosure and are provided as illustrative examples of the disclosure, not as restrictive descriptions. It is submitted with the understanding that they will not be used to limit the scope or meaning of the claims. In addition, in the detailed description, it can be seen that the description provides illustrative examples, and the various features are grouped together in various implementations for the purpose of streamlining the disclosure. The method of disclosure is not to be interpreted as reflecting an intention that the claimed subject matter requires more features than are expressly recited in each claim. Rather, as the claims reflect, inventive subject matter lies in less than all features of a single disclosed configuration or operation. The claims are hereby incorporated into the detailed description, with each claim standing on its own as a separately claimed subject matter.

### List of references

- 1: Marine vessel
- 3: Hull
- 5: Outer surface of hull
- 7: Inboard volume of hull
- 9: Stern
- 11: Bow
- 13: Inboard housing
- 15: Body of water
- 17: Surface of body of water
- 19: Water sample device
- 21: Water testing part
- 23: Moon-Pool
- 25: Set of wheels
- 27: Bottom of hull
- 29: Base of hull
- 31: Inner wall of moon-pool
- 33: Second water sample device
- 35: Third water sample device
- 37: Fourth water sample device
- 39: Fifth water sample device
- 41: Sixth water sample device
- 43: Control unit
- 45: Controller
- 101: Control system
- 102: Water sample system
- 103: Marine vessel
- 105: First water sample device
- 107: Second water sample device
- 109: Subsequent water sample device
- 111: Predefined model data
- 112: Controller
- 113: Water testing part
- 115: Sensor
- 117: Pattern recognition system
- 119: Test protocol
- 121: Navigation system
- 123: Navigation information
- 125: Communication system
- 127: Internet/cloud
- 129: User terminal
- 131: Control unit
- 133: Propulsion system
- 201: Method of obtaining a water sample
- A: Direction of movement

## Claims

1. A method of obtaining samples from a body of water by a marine vessel in a body of water, the marine vessel comprising an electronic device, the method comprising:
- obtaining a predefined model data of a first water sample device
- generating a first primary control signal for the first water sample device and performing a first movement of a first testing part in a first position relative to the the body of water,
- receiving a first sensor data from the first water sample device,
- generating first comparison data where the first sensor data is compared with the predefined model data of the first water sample device,
- generating a second primary control signal to the first water sample device based on the first comparison, and
- performing a second movement of the first water testing part relative to the body of water.

2. A method in accordance with claim 1, wherein the first comparison data is used to update the predefined model data of the first water sample device.

3. A method in accordance with any of the preceding claims, wherein the first comparison data is configured to determine the second movement of the first water testing part relative to the body of water.

4. A method in accordance with any of the preceding claims, wherein the second movement of the first water sample device retrieves the first water testing part from the body of water.

5. A method in accordance with any of the preceding claims, wherein the second movement of the first water testing part is to perform a second movement of the first testing device to a second position relative to the body of water, where the second position is different from the first position, receiving a second sensor data from the first water sample device.

6. A method in accordance with any of the preceding claims, wherein the method further comprises providing a predefined model data of a second water sample device, generating a first control signal for the second water sample device and performing a first movement of a second testing part in a first position in the body of water, receiving a first sensor data from the second water sample device, generating first comparison data where the first sensor data is compared with the predefined model data of the second water sample device, generating a second primary control signal to the second water sample device based on the first comparison, performing a second movement of the second water testing part relative to the body of water.

7. A method in accordance with any of the preceding claims, wherein the method further comprises generating a first secondary control signal for the marine vessel, where the first secondary control signal is used to perform a positional movement of the marine vessel relative to the body of water.

8. A method in accordance with any of the preceding claims, wherein the model data comprises a first data set comprising a testing procedure (and thereby a first movement and a second movement of the testing part, and/or positional movement of the marine vessel) of a first testing device, second testing device and/or any subsequent testing device or a second data set comprising predefined model data for sensor data comparison.

9. A method in accordance with any of the preceding claims, wherein the first sensor data is sensor data from one or more of conductivity sensor, temperature sensor, pressure sensor, dissolved oxygen sensor, fluorescence sensor, ORP, turbidity sensor, Voltage sensor, Photosynthetically Active Radiation (PAR) sensor, water sampler sensor, sediment core sampler sensor, Multibeam sonar sensor, Echo sounder sensor, Underwater camera sensor, Manta net sensor, Micro plastic filter pump sensor, Metal detector, Microphone sensor, optical sensor, odour sensor, rain sensor, light sensor, weight sensor, and/or cooling sensor.

10. A method in accordance with any of the preceding claims, wherein the first water sample device, the second water sample device or any subsequent water sample device performs a measurement where the first sensor data represents attributes of at least part of the body of water.

11. A method in accordance with any of the preceding claims, wherein the first water sample device, the second water sample device or any subsequent water sample device collects a part of the body water in one or more sample container.

12. A method in accordance with any of the preceding claims, wherein the performance of the first movement of a first testing part is in a first position in the body of water.

13. A method for controlling a marine vessel, wherein the method comprises providing a first control data, where the control data is compared to a predefined set of control data, and if the control data is in accordance with the predefined set of control data the method of claims 1-12 is initiated.

14. A marine vessel configured for obtaining samples from a body of water, the marine vessel comprising a processor, memory and an interface, wherein the processor is configured to
- providing predefined model data of a first water sample device
- generating a first primary control signal for the first water sample device and performing a first movement of a first testing part in a first position relative tothe body of water,
- receiving a first sensor data from the first water sample device,
- generating first comparison data where the first sensor data is compared with the predefined model data of the first water sample device,
- generating a second primary control signal to the first water sample device based on the first comparison, and
- performing a second movement of the first water testing part relative to the body of water.
